# EUROPEAN PATENT APPLICATION

(11) **EP 1 852 053 A1**
(43) Date of publication of application: **07.11.2007**
(21) Application number: 06714528.4
(22) Date of filing: 24.02.2006
(51) Int. Cl.: A61B 1/00

(54) **AUXILIARY TOOL FOR LARGE INTESTINE ENDOSCOPY**

(30) Priority: 24.02.2005 JP 2005049805; 09.09.2005 JP 2005263071
(71) Applicant: Japanostick Co., Ltd, Tokyo 111-0041 (JP)
(72) Inventor: HATTORI, Ryoji, Tokyo 113-0024 (JP)
(74) Representative: Chettle, Adrian John
(86) International application number: PCT/JP2006/303389
(87) International publication number: WO 2006/090822

(57) **Abstract**

[Problem]

A colonoscopic auxiliary tool is offered which permits an inspecting doctor to perform colonoscopic examinations easily without requiring long-term training.

[Means for Resolution]

A colonoscopic auxiliary tool 10 for use with a colonoscope during a colonoscopic examination. The tool has a tubular body portion 11 disposed inside the rectoanal region 12 and a suction-and-depressurization portion 16 mounted in the tubular body portion 11. The colonoscope 13 is passed through the tubular body portion 11. The suction-and-depressurization portion 16 can bring the inner wall 15 of the large intestine into intimate contact with an outer surface portion 56 of a cylindrical portion of the scope by depressurizing the inside 57 of the large intestine from the rectoanal region 12.

## Description

### TECHNICAL FIELD

The present invention relates to an auxiliary tool for colonoscopic examinations and, more particularly, to an auxiliary tool which is used for colonoscopic examinations, facilitates insertion of a colonoscope into the large intestine, traction, shortening, and straightening of the intestinal canal, and permits examinations to be performed safely without requiring long-term training.

### BACKGROUND ART

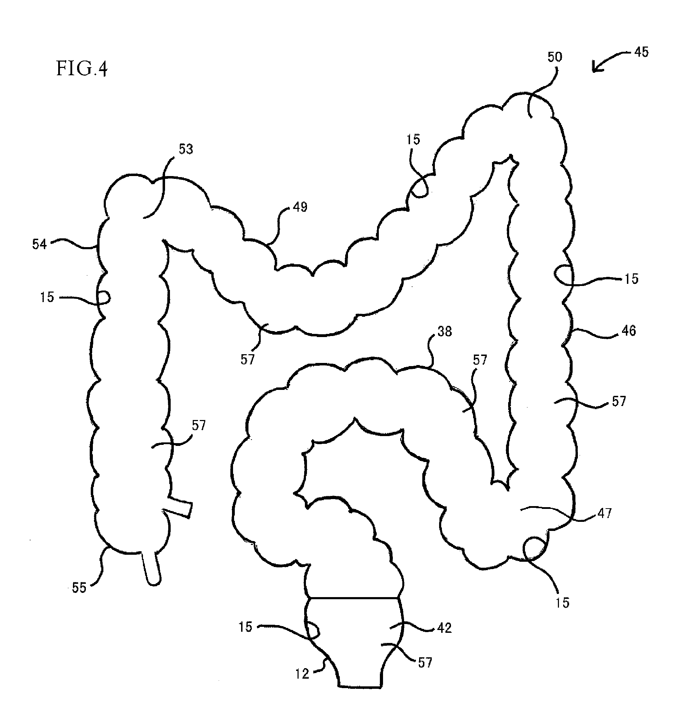

Hitherto, colonoscopes used for colonoscopic examinations have used front scope mounted type colonoscopes provided with a suction port portion and an observation lens at the front-end portion of such a colonoscope.
Incidentally, as shown in Fig. 4, the inside of the human large intestine is made up of those portions (i.e., rectal region 42, descending colon region 46, and ascending colon region 54) (hereinafter referred to as the fixed intestines) which are held by surrounding muscle fibers such as peritoneums and guts and those portions (hereinafter referred to as the free intestines) which are not held by the surrounding muscle fibers such as peritoneums or guts) (i.e. , sigmoid colon region 38 and transverse colon region 49). Large flexure regions or flexural regions known as hepatic flexure region 53, splenic flexure region 50, and sigmoid colon-descending colon junction region 47 are formed between the fixed and free intestines.

When a colonoscope is inserted from the rectoanal region of the examinee such that the scope reaches the descending colon region 46 acting as the fixed intestine from the sigmoid colon region 38 acting as the free intestine, only if the colonoscope is pushed in, the intestinal wall of the sigmoid colon portion 38 is simply stretched in the direction of insertion of the colonoscope because the sigmoid colon region 38 is formed like a hairpin curve bent sharply relative to the direction of the anal region. It is difficult to insert the scope. In some cases, manipulation of the scope may involve an accident consisting of perforation through the intestinal wall.
Such scopes are also used for examinations of esophagus, stomach, and duodenum. The esophagus, stomach, and duodenum are internal organs which are all simple in shape and structure. The doctor can easily insert a scope into them and perform examinations.
However, unlike examinations with gastrofiberscopes as described above, it is necessary for colonoscopy to pass into the complex large intestine made up of the rectal region 42 and so on which are fixed intestines held by surrounding muscle fibers such as peritoneums and guts, sigmoid colon region 38 and so on which are free intestines not held by the surrounding muscle fibers such as peritoneums or guts, and sigmoid colon-descending colon junction region 47 and so on formed between the fixed and free intestines and bent greatly like a hairpin curve.

Accordingly, from the past, in a case where the large intestine is colonoscopically examined with a colonoscope, a doctor who conducts examinations is required to receive training over a long term to insert the colonoscope into the large intestine safely and smoothly. Although about 2 million colonoscopic examinations are carried out per year in this nation, it is unlikely that everyone can perform colonoscopic examinations. Also, there are insufficient educational facilities for performing the above-described colonoscopic examinations. In the worst case, there has been the problem that the intestinal wall is injured during examination. An accident in which a perforation is formed may occur.

In this case, from the viewpoint to eliminate the difficulty of a work for inserting the above-described colonoscope, a method of insertion has been proposed which consists of repeatedly pulling back the free intestine portions together with the colonoscope toward the anus to fold them up, holding the free intestine portions, which would normally vary in shape freely, by folding up them, and inserting the colonoscope into the fixed intestines while the scope extends continuously and straight to the fixed intestines, rather than the method consisting of sucking the inner walls of the free intestines by a negative pressure in a sucking portion formed at the front-end portion of the scope such that the inner walls are stuck to the scope and pushing in the colonoscope into the large intestine.

From this viewpoint, from the past, a colonoscope has been proposed which is designed to draw in the air inside the intestinal canal from a suction port that is formed in the front of the scope and acting also as a clamp port for forming a negative pressure, to draw the inner walls of the free intestines to the scope, to pull back the scope, to perform dragging by making use of folding of the sigmoid colon region and transverse colon region that are the free intestines such that the free intestines can be folded like bellows (patent references 1 and 2).
Meanwhile, in the past, techniques for facilitating inserting a colonoscope by the use of a conoloscopic auxiliary tool have been proposed (patent references 3 and 4).
Patent reference 1: WO94/10896
Patent reference 2: JP-A-2002-125921
Patent reference 3: JP-A-64-42001
Patent reference 4: JP-A-01-227737

### DISCLOSURE OF THE INVENTION

### PROBLEM THAT THE INVENTION IS TO SOLVE

In this case, in order to obtain a negative pressure necessary to adhesively capture the internal wall of the large intestine over the whole region of the colonoscope, a strong attractive force is necessary. Such a conventional colonoscope is so constructed that the gas inside the large intestine is drawn in only by the suction port portion formed at the front-end portion of the colonoscope, so that shortening and traction are carried out relying on folding of the large intestine. Consequently, only the mucous layer and tunica submucosa out of the soft mucosae of the intestinal canal consisting of mucous layer, tunica submucosa, panniculus carnosus, and serous membrane layer are drawn in by the suction port portion. Since an observational lens portion which is mounted at the front-end portion of the colonoscope and adjacent to the suction port is covered by the mucous layer of the intestinal canal, a field of view necessary for colonoscopic examination cannot be secured. It has been difficult to insert the scope further.

In this case, the state of suction from the suction port portion is stopped to secure a field of view. Where the negative pressure is relieved, the adhesion between the scope and the inner wall of the large intestine become insufficient. This makes it impossible to fold the large intestine.
As a result, when shortening and traction using folding of the free intestines as described above are performed in colonoscopy, there are undesirable situations that long-term training is necessary and that masterly skill relying on intuition is required.

Furthermore, in the conventional colonoscope, the suction port portion is formed only at the front-end portion of the scope as described previously and so the inner wall of the large intestine is sucked to only the front-end portion of the scope. Adhesion of the scope to the inner wall of the large intestine is insufficient and, therefore, during the work for folding, the inner wall of the large intestine often disengages from the side surface portion of the scope.
As a result, the work for sucking the inner wall of the large intestine and the work for folding it must be repeated each time this situation occurs. This prolongs the time for which the colonoscope is inserted in the examinee. There is an undesirable situation that pain is given to the examinee.

That is, in the conventional colonoscopic examination described above, the large intestine which is in intimate contact with the side surface of the front-end portion of the colonoscope and has been folded easily disengages during the folding work. Therefore, in order to stick the large intestine to the colonoscope and fold the intestine reliably, it is necessary to repeat the manipulation for moving back and forth the scope plural times. The examinee's rectoanal region is frictionally worn away by the manipulation for moving back and forth the scope. In some cases, pain may occur.
Further, the technique described in patent reference 3 is intended to stick and straighten only the sigmoid colon region. It has been impossible to stick the transverse colon region that is a free intestine other than the sigmoid colon region. Therefore, it is impossible to insert the colonoscope smoothly and safely up into the deepest portion of the ascending colon region via the transverse colon region. It has been impossible to assist the scope until it reaches the cecal region.

The technique described in patent reference 4 performs suction by means of suction means fitted only at the front-end portion of an auxiliary tool for insertion. Therefore, only the mucous layer and tunica submucosa out of the soft mucous membranes of the intestinal canal are pulled in by the suction port portion described above in the same way as in the prior art. The observational lens portion mounted at the front-end portion of the scope is covered. Therefore, it is impossible to secure a field of view necessary for colonoscopic examination. It has been difficult to move the scope into the cavity portion of the large intestine.

Furthermore, the inspection auxiliary tool associated with the above-described patent reference 4 does not operate to bring the inner wall of the large intestine into intimate contact with the whole of the outer surface of the cylindrical portion of the scope. Rather, the tool is used only for straightening of the sigmoid colon region and, therefore, it has been impossible to stick the intestines at the transverse colon region that is a free intestine other than the sigmoid colon region. Consequently, it has been difficult to assist insertion of the colonoscope until it reaches the cecal region.

Accordingly, the problem to be solved by the present invention is to provide an auxiliary tool which is for use in colonoscopic examination and which gives a large frictional resistive force between the colonoscope and the inner wall of the large intestine while securing a field of view of the scope during insertion into the large intestine, reliably carries out folding of the free intestines such that an inspecting doctor can insert the colonoscope smoothly and safely from the sigmoid colon region (a free intestine) to the deepest portion of the ascending colon region via the transverse colon region (also a free intestine) without requiring long-term training, and can perform colonoscopic examinations safely and easily.

### MEANS FOR SOLVING THE PROBLEMS

To solve such a technical problem, the invention set forth in claim 1 is a colonoscopic auxiliary tool which is used with a colonoscope during a colonosconic examination and which is characterized in that it has a tubular body portion disposed in the rectoanal region and a suction-and-depressurization portion mounted in the tubular body portion. The colonoscope is inserted into the tubular body portion. The suction-and-depressurization portion depressurizes the inside of the large intestine from the rectoanal region such that the inner wall of the large intestine can be brought into intimate contact with the outer surface portion of the cylindrical portion of the colonoscope.
Accordingly, in the invention set forth in claim 1, the colonoscopic auxiliary tool is so used that the colonoscope is inserted in the tubular body portion under the condition where the tubular body portion is disposed inside the rectoanal region.

The invention set forth in claim 2 is characterized in that the suction-and-depressurization portion has a plurality of hole portions formed in the tubular body portion and a depressurization mechanism capable of forming a negative pressure in the large intestine by drawing in the air inside the large intestine from the hole portions via the plural hole portions. The depressurization mechanism has negative pressure supply passages in communication with the plural hole portions and a negative pressure supply portion capable of supplying the negative pressure via the negative pressure supply passages.
Accordingly, in the invention set forth in claim 2, the negative pressure is supplied from the negative pressure supply portion. A negative pressure can be formed inside the large intestine because the air inside the large intestine is drawn in from the plural hole portions.

The invention set forth in claim 3 is characterized in that a lubricant supply portion capable of supplying a lubricant into the tubular body portion such that the scope is smoothly inserted into the tubular body portion is formed in the tubular body portion.
Accordingly, in the invention set forth in claim 3, the lubricant is supplied into the tubular body portion, permitting the colonoscope to be smoothly inserted into the tubular body portion.

The invention set forth in claim 4 is characterized in that an air inflow cutoff portion capable of preventing inflow of air into the tubular body portion from the outside is mounted in the base portion side of the tubular body portion, the cutoff portion being pressed against the inserted colonoscope.
Accordingly, in the invention set forth in claim 4, the tubular body portion is so formed that inflow of air from outside the anus toward the base portion side is prevented by the air inflow cutoff portion.

The invention set forth in claim 5 is characterized in that it has a holding portion capable of holding the tubular body portion into the rectoanal region.
Accordingly, in the invention set forth in claim 5, the tubular body portion is held within the rectoanal region by the holding portion.

The invention set forth in claim 6 is characterized in that the holding portion has an expansible portion mounted outside the tubular body portion and a fluid supply portion capable of supplying a fluid into the expansible portion that can swell outwardly of the tubular body portion by being supplied with the fluid.
Accordingly, the invention set forth in claim 6 is so configured that the holding portion mounted outside the tubular body portion swells outwardly by being supplied with the fluid from the fluid supply portion.

The invention set forth in claim 7 is characterized in that the expansible portion is made of a balloon body which is mounted at the front-end portion of the tubular body portion and which swells out by being supplied with the fluid.
Accordingly, in the invention set forth in claim 7, the balloon body is so formed that it swells out by being supplied with the fluid.

The invention set forth in claim 8 is characterized in that the balloon body is formed along the whole periphery of the tubular body portion.
Accordingly, in the invention set forth in claim 8, the balloon body is so formed that it abuts into the examinee's rectoanal region over the whole periphery of the tubular body portion.

The invention set forth in claim 9 is characterized in that holding elements capable of supporting the tubular body portion outside the anal region are mounted on a side of the base portion of the tubular body portion.
Accordingly, in the invention set forth in claim 9, the tubular body portion is so held that the tubular body portion does not come off outside the examinee's anal region.

### ADVANTAGES OF THE INVENTION

Since the present invention is configured as described above, the invention yields the following advantages.
According to the invention set forth in claim 1, the suction-and-depressurization portion performs suction and depressurization in the rectoanal region, unlike the prior-art colonoscope, i.e., different from the technique consisting of performing suction from suction hole portions formed in a front-end portion of a scope inserted into the large intestine to stick the inner wall of the large intestine to the colonoscope. The inner wall of the large intestine is stuck to the whole side surface portion of the scope along the direction of length of the side surface portion of the scope.

The inner wall of the large intestine is stuck to the whole side surface portion of the scope rather than only to the front-end portion of the scope. The inner wall of the large intestine is not concentratively stuck to only the front-end portion of the scope. Therefore, the undesirable situation with the prior art, i.e., an objective lens disposed in a front-end portion of a scope is covered by the inner wall of the large intestine, whereby the field of view is lost, can be eliminated. Consequently, a work for inserting the scope can be performed safely while securing a field of view of the scope.

Furthermore, as described above, the inner wall of the large intestine is stuck to the whole side surface portion of the scope rather than only to the front-end portion of the scope. A large frictional resistive force is produced between the colonoscope and the inner wall of the large intestine. Where the free intestines are folded, the folding work can be performed certainly.
Therefore, the situation occurring in the prior-art colonoscope, i.e., insufficient adhesion of the inner wall of the large intestine to the scope due to suction only at the front-end portion of the scope may permit the inner wall of the large intestine once stuck to disengage from the scope during the folding work, can be effectively prevented.

As a result, the problem that during the folding work, whenever the inner wall of the large intestine disengages from the side surface portion of the scope, a work for sucking the inner wall of the large intestine and a work for folding must be repeated, resulting in prolongation of the insertion time of the colonoscope in the examinee and giving pain to the examinee, can be solved.

In addition, the state in which the large intestine is folded is held on the rectoanal region side by drawing in the large intestine folded by the folding work by means of the suction-and-depressurization portion mounted in the rectoanal region. The situation in which the inner wall of the large intestine once brought into intimate contact disengages can be prevented. The colonoscope can be inserted while securing a field of view of the front-end portion.

Further, unlike the prior-art colonoscopic auxiliary tool, the novel colonoscopic auxiliary tool is used not only for straightening of the sigmoid colon region. The inner walls of the free intestines are brought into intimate contact with the whole of the outer surface of the cylindrical portion of the scope. As a result, the sigmoid colon and transverse colon regions which are free intestines can be folded up by pulling back the colonoscope to the outside of the anus. Therefore, the colonoscope can be inserted smoothly and safely from the sigmoid colon region that is a free intestine up into the cecal region that is the deepest portion of the ascending colon region via the transverse colon region that is a free intestine similarly. Furthermore, in the invention set forth in claim 1, the tubular body portion which is so formed that the scope can be passed through the tubular body portion is disposed in the rectoanal region and so pain in the skin around the anal ring caused by friction on the colonoscope when it is moved back and forth through the rectoanal region can be prevented.

According to the invention set forth in claim 2, the suction-and-depressurization portion is provided with a plurality of hole portions formed in the tubular body portion and so the colonoscopic auxiliary tool can draw in the air inside the large intestine uniformly from the hole portions and form a uniform negative pressure inside the examinee's large intestine.

According to the invention set forth in claim 3, even where the colonoscope is moved back and forth through the tubular body portion, a lubricant is supplied between the tubular body portion and the colonoscope. The lubricant adhered to the colonoscope is supplied into the anal region by the colonoscope by the manipulation of the colonoscope for moving it back and forth and, therefore, the frictional force between the colonoscope and the anal region near the anal sphincter can be reduced by the lubricant supplied from the lubricant supply portion. The manipulation of the colonoscope for moving it back and forth can be done smoothly and easily.
Intrinsically, the mucous membrane of the large intestine itself is protected by a lubricant liquid known as mucus at all times. However, secretion of the mucus is insufficient near the anal sphincter that is at the boundary between the large intestine and the skin around the anal region and so insertion of the colonoscope can be facilitated by supply of the lubricant into the anal region.

According to the invention set forth in claim 4, the air inflow cutoff portion is formed on a side of the base portion of the colonoscopic auxiliary tool and so even during insertion of the colonoscope, inflow of air into the tubular body portion from the gap formed between the tubular body portion and the colonoscope is prevented. Depressurized state inside the large intestine can be maintained reliably.

According to the invention set forth in claim 5, even where the work for passing the colonoscope into the tubular body portion, inserting the scope into the large intestine, and pulling back the scope is done, the situation in which the tubular body portion disengages from the rectoanal region can be prevented. A colonoscopic examination can be performed smoothly.

According to the invention set forth in claim 6, the expansible portion is mounted outside the tubular body portion and so the tubular body portion is abutted against the inner wall of the large intestine of the rectoanal region by swelling the expansible portion while the tubular body portion has been inserted in the examinee's rectoanal region. It is assured that the tubular body portion can be held inside the rectoanal region.

According to the invention set forth in claim 7, the tubular body portion is abutted against the inner wall of the large intestine in the rectoanal region of the examinee by swelling the balloon body while the tubular body portion has been inserted in the examinee's rectoanal region. Thus, tubular body portion can be held more reliably and safely. Even where a manipulation for inserting or pulling out the colonoscope is performed, the situation in which the colonoscopic auxiliary tool comes off from the examinee's rectoanal region or shifts can be prevented.

According to the invention set forth in claim 8, the balloon body is abutted into the examinee's rectoanal region over the whole periphery of the tubular body portion. Therefore, the tubular body portion is held by the balloon body over its whole periphery. Disengagement of the tubular body portion from inside the rectoanal region can be prevented.
Furthermore, since the balloon body is formed over the whole periphery of the tubular body portion, inflow of air into the large intestine from the gap formed between the tubular body portion and the rectonanal region is prevented. It is assured that the depressurized state of the inside of the large intestine can be maintained.

According to the invention set forth in claim 9, the tubular body portion is supported by the holding elements and so even where the work for drawing in and folding the large intestine is repeated using the colonoscopic auxiliary tool, the tubular body portion can be prevented from coming off from the examinee's anal region. The work for folding up the large intestine can be carried out reliably.

### BEST MODE FOR CARRYING OUT THE INVENTION

A colonoscopic auxiliary tool 10 associated with the present invention is hereinafter described in detail based on embodiments shown in the accompanying drawings.

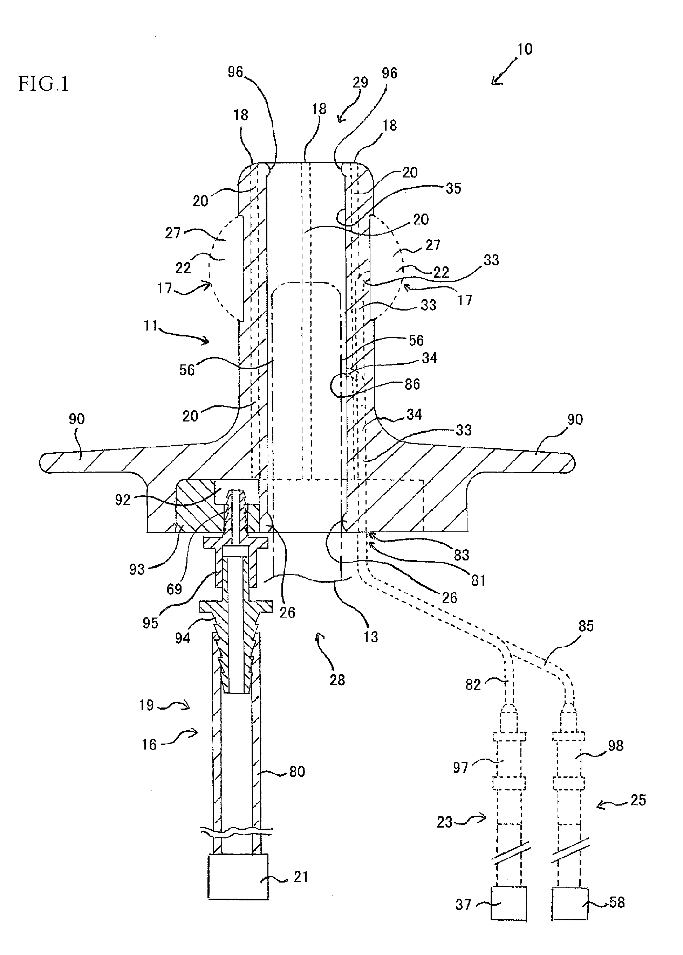

The colonoscopic auxiliary tool 10 associated with the present embodiment is a colonoscopic auxiliary tool used together with a colonoscope during colonoscopic examination as shown in Figs. 1-3, and has a tubular body portion 11 disposed in the rectoanal region 12 and a suction-and-depressurization portion 16 mounted in the tubular body portion 11. The colonoscope 13 is passed through the tubular body portion 11. The suction-and-depressurization portion 16 can depressurize the inside 57 of the large intestine from the rectoanal region 12 and bring the inner wall 15 of the large intestine into intimate contact with the outer surface portion 56 of the cylindrical portion of the colonoscope 13.

The suction-and-depressurization portion 16 has a plurality of hole portions 18 formed in the tubular body portion 11 and a depressurization mechanism 19 capable of drawing in air in the inside 57 of the large intestine from the hole portions 18 via the plural hole portions 18 to form a negative pressure in the inside 57 of the large intestine. The depressurization mechanism 19 has negative pressure supply passages 20 in communication with the plural hole portions 18 and a negative pressure supply portion 21 capable of supplying the negative pressure via the negative pressure supply passages 20.

Furthermore, as shown in Fig. 1, the tubular body portion 11 is provided with a lubricant supply portion 25 capable of supplying a lubricant into the tubular body portion 11 such that the scope is smoothly inserted into the tubular body portion 11.

Further, an air inflow cutoff portion 26 capable of preventing inflow of air from the outside into the tubular body portion 11 is mounted on a side of a base portion 28 of the tubular body portion 11 and pressed against the colonoscope 13 that is inserted.
In addition, there is provided a holding portion 17 capable of holding the tubular body portion 11 inside the rectoanal region 12.

Furthermore, the holding portion 17 has an expansible portion 22 mounted outside the tubular body portion 11 and a fluid supply portion 23 capable of supplying a fluid into the expansible portion 22, which can be swollen outwardly of the tubular body portion 11 by being supplied with the fluid.

The expansible portion 22 is made of a balloon body 27 that is mounted in a front-end portion 29 of the tubular body portion 11. The balloon body swells when a fluid is supplied into it.
The balloon body 27 is mounted over the whole periphery of the tubular body portion 11.
Holding elements capable of supporting the tubular body portion outside the anal region are mounted on a base portion side of the tubular body portion.

### EMBODIMENTS

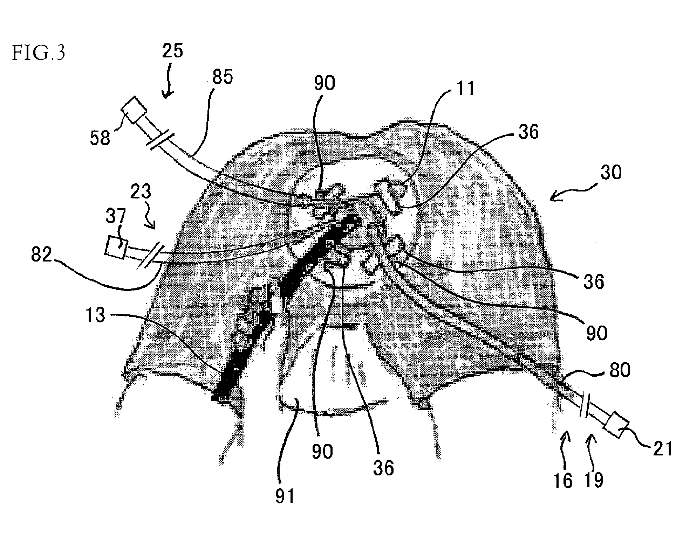

As shown in Fig. 3, the colonoscopic auxiliary tool 10 associated with the present embodiment is used when the colonoscope 13 is inserted into the rectoanal region 12 of an examinee 30 and a colonoscopic examination is performed.
In Fig. 3, the examinee 30 is placed in a prone position on an examination table (not shown) . The colonoscope 13 is used under the illustrated state, which is illustrated now. A pillow 91 assuming a horseshoe shape within a plane at the positions of the pubic bone portion and bone spur in the foregut provides a cover over the front surface portion and side surface portion of the hypogastric region. The pillow is placed between the examination table and the examinee. The colonoscopic auxiliary tool 10 is installed while raising the pubic bone portion and bone spur in the foregut of the examinee 30 upward.

As shown in Figs. 1, 2, and 4, the colonoscopic auxiliary tool 10 is disposed in the rectoanal region 12, and has the tubular body portion 11 through which the colonoscope 13 is passed and the suction-and-depressurization portion 16 mounted in the tubular body portion 11. The suction-and-depressurization portion can depressurize the inside 57 of the large intestine from the rectoanal region 12 and bring the inner wall 15 of the large intestine into intimate contact with the outer surface portion 56 of the cylindrical portion of the scope.

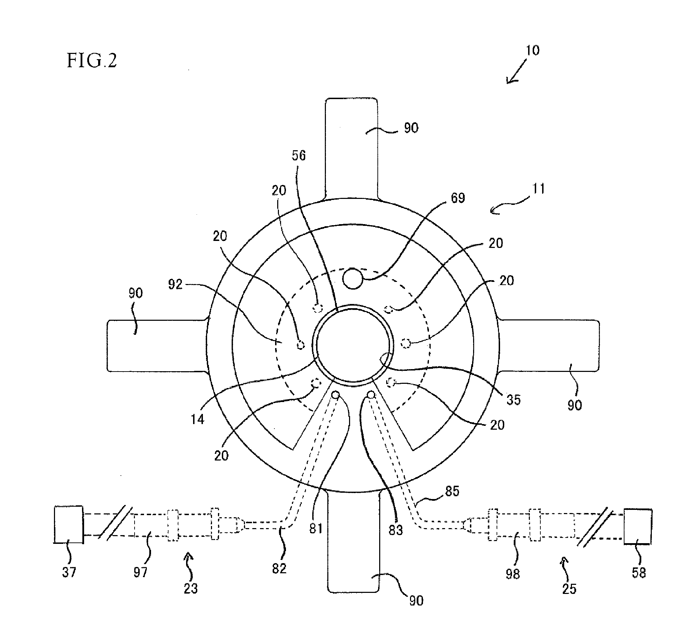

As shown in Figs. 1 and 2, the tubular body portion 11 is made of a silicone material. The body portion can be discarded after use to avoid contamination with pathogenic bacteria such as AIDS.
The tubular body portion 11 is shaped as a short cylindrical form. The diametrical dimension of the body portion is so set that it can be inserted into the rectoanal region 12 of the examinee 30. In the present embodiment, the tubular body portion 11 is so formed that its outside diameter is 25 mm. The tubular body portion 11 is so formed that its total length is 70 mm.

Since the tubular body portion 11 is made of a silicone material as described above, the body portion has flexibility. The body portion can be bent in conformity with the shape of the rectoanal region 12 of the examinee 30. A substantially cylindrical cavity portion is formed inside the body portion.

The tubular body portion 11 is shaped cylindrically. The diameter of the internally formed cavity portion is set to a diametrical dimension that is slightly larger than the diameter of the colonoscope 13. In the present embodiment, the inside diametric dimension is set to 15 mm on the assumption that the diametric dimension of the colonoscope 13 is 14 mm.

In the present embodiment, the tubular body portion 11 is made of a silicone material. The tubular body portion 11 is made of a pliable plastic material or a metal.

As shown in Fig. 1, holding elements 90, 90, 90, 90 capable of supporting the tubular body portion 11 outside the anal region are mounted on a side of the base portion 28 of the tubular body portion 11.
The holding elements 90, 90, 90, 90 are shaped rectangularly within a plane. The four elements protrude sideways like jaws on a side of the base portion 28 of the tubular body portion 11.
The outside diametric dimension of the holding elements 90, 90, 90, 90 is set to a diametrical dimension that is larger than the outside diameter of the rectoanal region 12 of the examinee 30. Even where the tubular body portion 11 is inserted in the rectoanal region 12 of the examinee 30, the tubular body portion 11 is held within the rectoanal region 12.
In use, as shown in Fig. 3, the holding elements 90, 90, 90, 90 are held to the skin of the examinee 30 around the anal region using viscous tape. The tubular body portion 11 is held stationary and prevented from moving. Under this condition, it is used.

As shown in Figs. 1 and 2, the suction-and-depressurization portion 16 has six hole portions 18 formed in the front-end portion 29 of the tubular body portion 11 and the depressurization mechanism 19 capable of drawing in air within the inside 57 of the large intestine from the hole portions 18 via the 6 hole portions 18 to form a negative pressure within the inside 57 of the large intestine.
The depressurization mechanism 19 has six negative pressure supply passages 20 in communication with the 6 hole portions 18 and the negative pressure supply portion 21 capable of supplying the negative pressure via the negative pressure supply passages 20.

As shown in Fig. 1, the negative pressure supply portion 21 is made of an appropriate compressor, and the negative pressure supply passages 20 are formed in the tubular body portion 11. The negative pressure supply passages 20 and the compressor are connected by hoses 80 capable of supplying an appropriate negative pressure.

As shown in Fig. 1, a negative pressure chamber 92 formed so as to be capable of combining the 6 negative pressure supply passages 20 into one is formed on a side of the base portion 28 of the tubular body portion 11. A cover portion 93 formed so as to be capable of closing the negative pressure chamber 92 is mounted in an end portion on a side of the base portion 28 of the tubular body portion 11. In use, the negative pressure chamber 92 is formed by mounting the cover portion 93 to a side end portion of the base portion 28 of the tubular body portion 11.
A negative pressure supply hole portion 69 formed so as to be inserted into the negative pressure chamber 92 is formed in the cover portion 93.

As shown in Fig. 1, the hoses 80 are connected via two adapters 94 and 95 (larger one and smaller one) which can be inserted and held in the negative pressure supply hole portion 69.
Each of the adapters 94 and 95 is made of a cylindrically shaped portion and a plurality of conic engagement elements having different system dimensions.
The hoses 80 are connected with the adapter 94 by coupling together the cylindrically shaped portions of the adapters 94, 94 and inserting the engaging element portion of the adapter 95 of the larger diameter into the hoses 80. The adapter 95 can be connected with the negative pressure supply hole portion 69 by inserting the engagement element portion of the adapter 95 of the smaller diameter into the negative pressure supply hole portion 69.

Each of the negative pressure supply passages 20 is formed between the negative pressure chamber 92 and each hole portion 18. The negative pressure supply passages 20 are formed along the axial direction within the tubular wall of the tubular body portion 11 and are six in number. The negative pressure supply passages 20 are in communication with six hole portions 18 opening into the front-end portion 29 of the tubular body portion 11.

Therefore, as shown in Figs. 1, 2, and 4, the negative pressure supplied into the negative pressure supply passages 20 from the compressor that is a negative pressure supply source is supplied into the large intestine via the hole portions 18. As a result, the inside of the large intestine is depressurized and the inner wall 15 of the large intestine is drawn in.
The inner wall 15 of the large intestine is brought into intimate contact with the outer surface portion 56 of the cylindrical portion of the colonoscope by depressurization of the inside 57 of the large intestine. Of the large intestine, the free intestines are folded like bellows and shortened by dragging the colonoscope 13 toward the anal region 12 of the large intestine.

As shown in Figs. 1 and 2, the holding portion 17 capable of holding the tubular body portion 11 is mounted in the front-end portion 29 of the tubular body portion 11. The holding portion 17 has the expansible portion 22 mounted outside the tubular body portion 11 and the fluid supply portion 23 capable of supplying a fluid into the expansible portion 22, which can swell outwardly of the tubular body portion 11 by being supplied with the fluid.
The expansible portion 22 is made of the balloon body 27 which is mounted in the front-end portion 29 of the tubular body portion 11 and swells out by being supplied with the fluid.

As shown in Fig. 1, the fluid supply portion 23 is made up of a fluid supply source 37, a fluid supply hole portion 81 formed at an end portion on a side of the base portion 28 of the tubular body portion 11, a hose 82 disposed between the fluid supply hole portion 81 and the fluid supply source 37 and being capable of supplying an appropriate fluid, and a single fluid supply passage 33 formed along the axial direction within the tubular wall and between the fluid supply hole portion 81 and the balloon body 27.
A check valve 97 is mounted between the hose 82 and the fluid supply source 37 to prevent reverse flow of the fluid supplied from the fluid supply source 37.

The balloon body 27 is made of a synthetic resin and shaped like a bag, and is firmly mounted to the whole periphery of the outer peripheral wall of the front-end portion 29 of the tubular body portion 11.
Before the tubular body portion 11 is inserted into the rectoanal region 12 of the examinee 30, the fluid inside the balloon body 27 has been discharged. The bag-like balloon body 27 in a shrunk state is mounted in the tubular body portion 11.

Where the tubular body portion 11 has been completely inserted in the rectoanal region 12 of the examinee 30, a fluid is supplied into the balloon boy 27 from the fluid supply source 37 via the fluid supply passage 33. The balloon body is filled with the fluid supplied from the fluid supply portion 23, so that the balloon body 27 becomes swollen out like a bag.

In the present embodiment, the fluid supplied into the balloon body 27 is water. A fluid which does not adversely affect the human body even where the balloon body 27 breaks up and the fluid flows out into the inside 57 of the large intestine is used as the above-described fluid.

The inside of the balloon body 27 is filled with the fluid by supply of the fluid from the fluid supply portion 23. The balloon body 27 is swollen inside the rectoanal region 12 of the examinee 30. Since the anal sphincter of the examinee 30 is pressed against the tubular body portion 11 in the rectoanal region 12 of the examinee 30, the tubular body portion 11 can be held inside the rectoanal region 12.

The balloon body 27 of the tubular body portion 11 which has been swollen out like a bag as described above is pressed against the inner wall 15 of the large intestine inside the rectoanal region 12 of the examinee 30. The tubular body portion 11 is held inside the rectoanal region 12.

As shown in Figs. 1 and 2, the tubular body portion 11 is provided with the lubricant supply portion 25 capable of supplying a lubricant into the tubular body portion 11 such that the colonoscope 13 is smoothly inserted into the tubular body portion 11.

The lubricant supply portion 25 is made up of a lubricant supply source 58, a lubricant supply hole portion 83 formed at an end portion on a side of the base portion 28 of the tubular body portion 11, a hose 85 disposed between the lubricant supply source 83 and the lubricant supply source 58 and being capable of supplying an appropriate fluid, an opening portion 86 opening into an internal cavity of the tubular body portion 11 in the tubular wall, and a single lubricant supply passage 34 formed between the opening portion 86 and the lubricant supply hole portion 83 and along the direction of the length of the tubular body portion 11 within the tubular wall.
A check valve 98 is mounted between the hose 85 and the lubricant supply source 58 to prevent reverse flow of the lubricant supplied from the lubricant supply source 58.

Therefore, the lubricant supply portion is so configured that a lubricant passes from the lubricant supply source 58 to the lubricant supply hole portion 83 via the hose 85 and is supplied from the lubricant supply passage 34 to the inner side surface of the tubular wall via the opening portion 86.

The lubricant is formed like jelly and facilitates a manipulation of the colonoscope 13 for moving it back and forth while maintaining the airtightness between the outer surface portion 56 of the cylindrical portion of the scope and the inner wall peripheral surface 35 and maintaining the negative pressure inside the large intestine.

Where the lubricant is sucked and becomes disappeared during the manipulation of the colonoscope 13 for moving it back and forth, the lubricant is additionally supplied from the lubricant supply source 58 to the cavity portion between the cylindrical portion 14 of the scope and the tubular body portion 11 via the lubricant supply passage 34. Consequently, the lubricant is always present on the inner peripheral wall surface 35 inside the tubular body portion 11.

As shown in Fig. 1, the air inflow cutoff portion 26 capable of preventing inflow of air into the tubular body portion 11 from the outside is mounted on a side of the base portion 28 of the tubular body portion 11 and pressed against the inserted colonoscope 13.
The air inflow cutoff portion 26 is formed over the whole periphery of the inside of an end portion of the base portion 28 such that where the colonoscope 14 is inserted inside an end portion of the base portion 28 of the tubular body portion 11, the air inflow cutoff portion 26 can be pressed against the outer peripheral surface portion of the colonoscope 14, thus maintaining the state of the negative pressure.

The air inflow cutoff portion 26 is molded integrally with the tubular body portion 11 and is brought into intimate contact with the outer surface portion 56 of the cylindrical portion of the scope. The inside 57 of the large intestine is depressurized. Thus, the air inflow cutoff portion prevents inflow of air into the rectoanal region 12 from the base portion 28.

Similarly, as shown in Fig. 1, an air inflow cutoff portion 96 is mounted on a side of the front-end portion 29 of the tubular body portion 11 and can be pressed against the inserted colonoscope 13 to prevent inflow of air into the tubular body portion 11 from the outside.
The air inflow cutoff portion 96 is formed over the whole periphery of the inside of an end portion of the front-end portion 29 of the tubular body portion 11 such that where the colonoscope 14 is inserted, the air inflow cutoff portion 96 can be pressed against the outer peripheral surface portion of the colonoscope 14, thus maintaining the state of the negative pressure.

The air inflow cutoff portion 96 is molded integrally with the tubular body portion 11 and is brought into intimate contact with the outer surface portion 56 of the cylindrical portion of the scope. The inside 57 of the large intestine is depressurized. Thus, the air inflow cutoff portion prevents inflow of air into the rectoanal region 12 from the front-end portion 29.

The operation of the colonoscopic auxiliary tool 10 associated with the present invention is hereinafter described in detail based on embodiments shown in the accompanying drawings.
As shown in Fig. 3, the colonoscopic auxiliary tool 10 is inserted into the rectoanal region 12 of the examinee 30 during colonoscopic examination. This facilitates insertion of the colonoscope 13. As shown in Fig. 22, the air within the inside 57 of the large intestine is drawn in by the colonoscopic auxiliary tool 10. This brings the inner wall 15 of the large intestine into intimate contact with the outer surface portion 56 of the colonoscope. The colonoscope 13 is dragged as shown in Fig. 23, so that the free intestines are folded like bellows. Consequently, the sigmoid colon region 38 that is a free intestine and arranged like a hairpin curve is shortened. This can straighten the sigmoid colon region 38, facilitating insertion of the colonoscope 13.

When the colonoscopic auxiliary tool 10 associated with the present embodiment is used, the colonoscope 13 has been previously inserted in the cavity portion formed in the tubular body portion 11 of the colonoscopic auxiliary tool 10.

Before the tubular body portion 11 is inserted into the rectoanal region 12, the balloon body 27 formed like a bag as described above is supplied with no fluid and so is in shrunk state. Therefore, the balloon body 27 is so shaped that it can be easily inserted into the rectoanal region 12 on the outer peripheral surface portion of the tubular body portion 11.

After the colonoscope 13 on which the conoloscopic auxiliary tool 10 has been mounted is inserted into the rectoanal region 12, water is supplied into the balloon body 27 from the fluid supply portion 23 via the fluid supply passage 33.
The supply of the liquid swells the balloon body 27, pushing against the anal sphincter of the examinee 30 from the inside. Thus, the balloon body is held inside the rectoanal region 12.

Then, as shown in Fig. 3, the holding elements 90, 90, 90, 90 mounted to the tubular body portion 11 are held to the skin of surroundings of the anal region of the examinee 30 with adhesive tape 36. Subsequently, a negative pressure is supplied from the negative pressure supply portion 21 into the large intestine via the negative pressure supply passages 20. As a result, the air inside the large intestine is drawn in via the 6 hole portions 18 formed in the front-end portion 29 of the colonoscopic auxiliary tool 10. A negative pressure is produced in the large intestine.

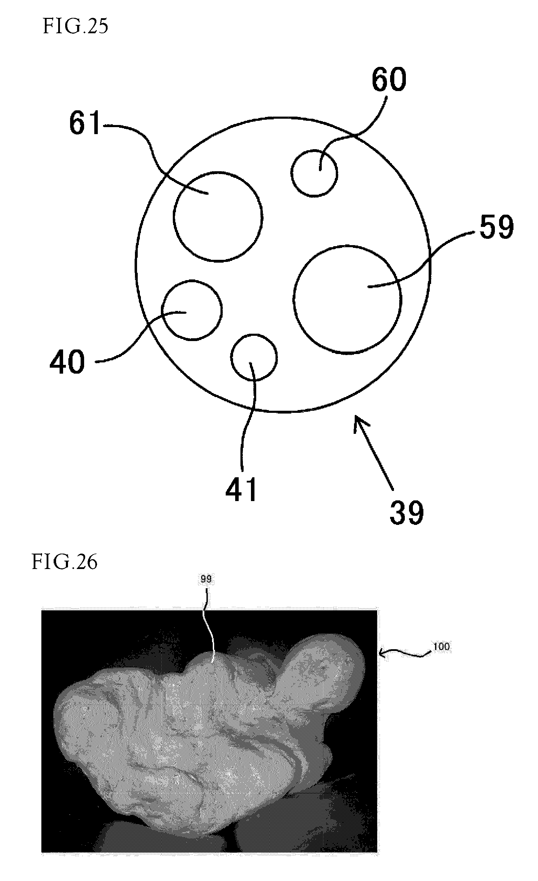

The colonoscope 13 used in the colonoscopic auxiliary tool 10 can be used irrespective of the kind such as fiberscope or electronic scope.
The colonoscope 13 used in the present embodiment has an air feeding hole 40, an air intake hole 41, an observational lens 59, a light guide 60, and a clamp port 61 in its front-end portion 39 as shown in Fig. 25.

Manipulation of the colonoscope 13 when the colonoscopic auxiliary tool 10 associated with the present embodiment under the state in which the examinee 30 is placed in a prone position is mounted is next described.
The colonoscope 13 inserted in the cavity portion of the tubular body portion 11 is inserted into the rectoanal region 12 of the examinee 30. The rectal region of the examinee 30 is observed with the colonoscope 13 and then the tubular body portion 11 of the colonoscopic auxiliary tool 10 is inserted into the rectoanal region 12 of the examinee 30.

In this case, the holding elements 90, 90, 90, 90 mounted to the tubular body portion 11 bear against the outside of the rectoanal region 12 of the examinee 30 and so excessive insertion is suppressed. Even a doctor who is not accustomed to examinations can insert the tubular body portion 11 into a given position in the rectoanal region 12 at all times. Insertion of the tubular body portion 11 can be performed precisely.

After observation of the rectal region of the examinee 30, the tubular body portion 11 is held inside the rectoanal region 12 of the examinee 30 as shown in Fig. 3. The colonoscope 13 is inserted into the cavity portion formed inside the tubular body portion 11, and is inserted into the rectoanal region 12 of the examinee 30.

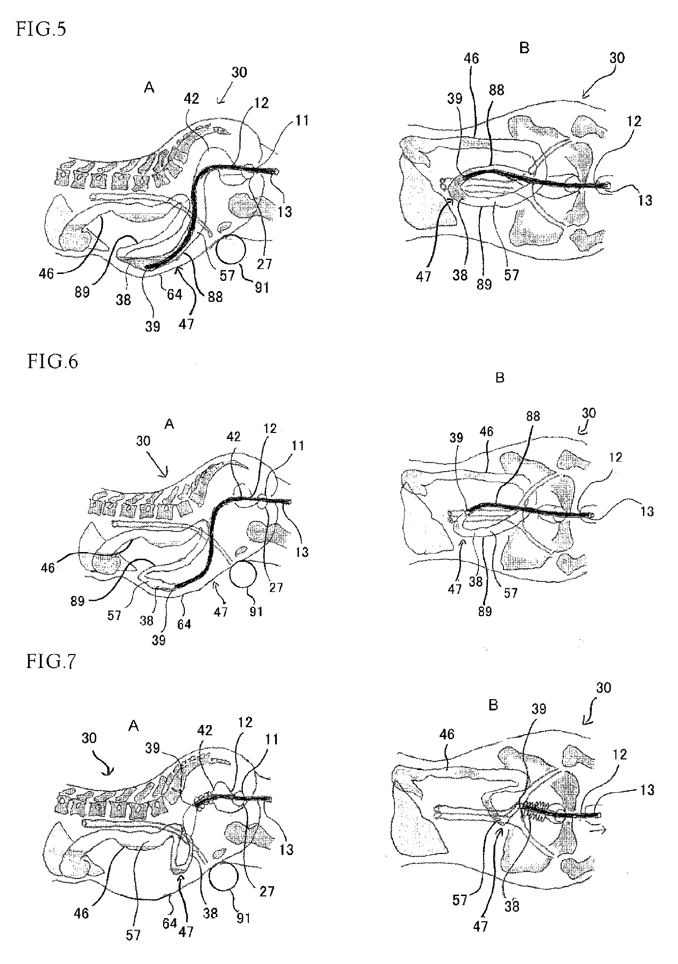

In the following, Figs. 5-11 show the state of progress of the colonoscope 13 inserted from the rectoanal region 12 until the scope reaches the descending colon region 46 from the sigmoid colon region 38. In each case, the examinee 30 is placed in a prone position. Figure A is a side elevation of the examinee 30. Figure B is a plan view showing the state in which an observation is made from the abdominal region of the examinee 30.
Accordingly, in figure A, the leftward direction indicates the head direction. The rightward direction indicates the leg direction. The downward direction indicates the abdominal region. In figure B, the leftward direction indicates the head direction. The rightward direction indicates the leg direction.

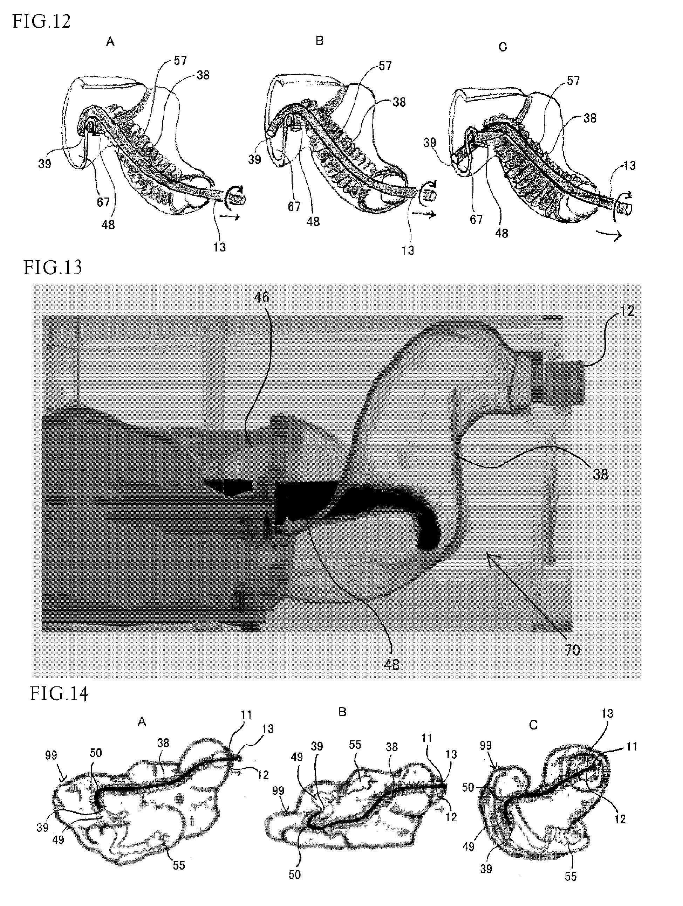

In Fig. 12, figure A shows the state in which the colonoscope has been rotated through 30 degrees to the right within the sigmoid colon region 38. Figure B shows the state in which the scope has been rotated through 45 degrees to the right. Figure C shows the state in which the scope has been rotated through 60 degrees to the right. Figs. 14-20 show plan views conceptually showing the state in which an observation is made from the abdominal region of the examinee 30.

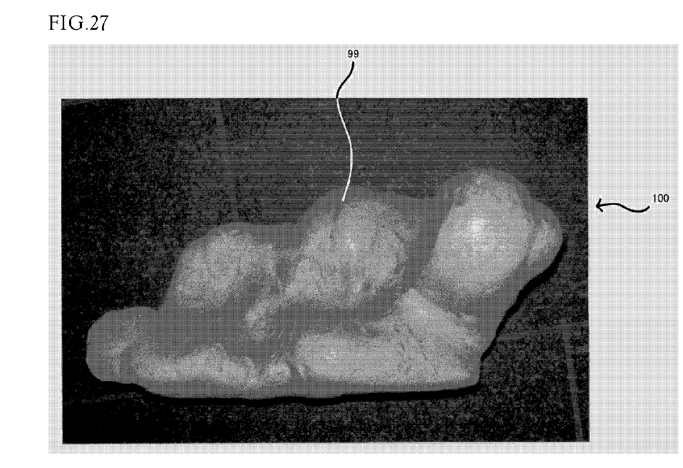

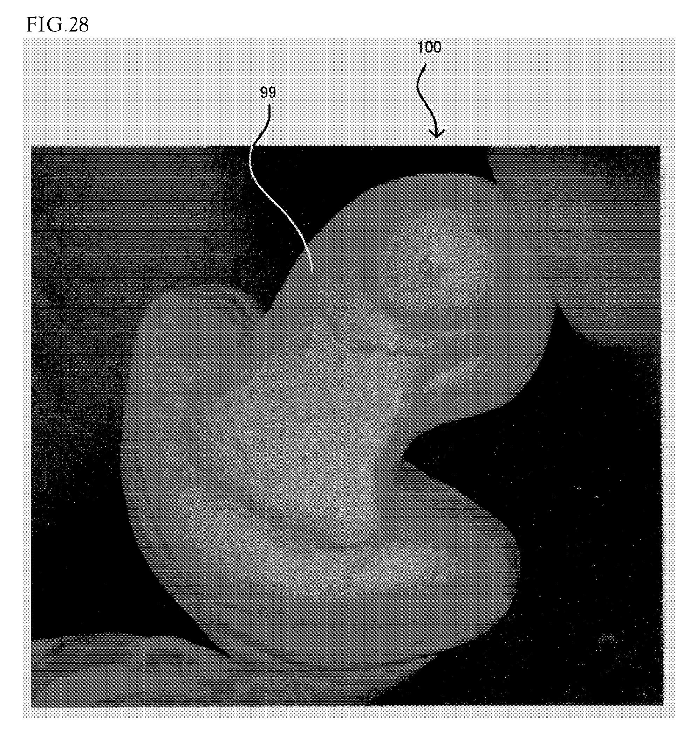

Figs. 26, 27, and 28 show a model 99 of gypsum formed based on an image of a maximum region where the free intestines (sigmoid colon region 38 and transverse colon region 49) inside the large intestine can move, the image being taken by CT scanning of the present inventor himself. Fig. 26 shows a side elevation of the state in which the left abdominal wall of the examinee who was placed in a prone position was pulled up 45 degrees. Fig. 27 shows a look-down view as taken from the rear surface of the examinee under similar conditions. Fig. 28 shows a view as viewed from the anal side of the examinee under similar conditions. Arrow 100 indicates the anal side.
Figs. 14-19 are side elevations under the condition in which the examinee is placed in a prone position and the left abdominal wall has been pulled up 45 degrees. Using the gypsum model 99, the state of progress until the colonoscope 13 reaches the cecal region 55 via the transverse colon region 49 is shown. In each case, the examinee 30 was placed in a prone position.
Figure A is a left side elevation of the examinee owing to the above-referenced Fig. 26. Figure B is a look-down view showing the state in which an observation was made from the rear surface of the examinee owing to the above-referenced Fig. 27. Figure C shows a view of the anal side, showing the state in which an observation was made from the anal side of the examinee owing to the above-referenced Fig. 28.

A process consisting of inserting the colonoscope 13 from the rectoanal region 12, inserting the scope into the descending colon region 46 that is a fixed intestine via the sigmoid colon region 38 that is a free intestine, and going into the splenic flexure region 50 that is the deepest portion of the descending colon region 46 is described in the following.
As shown in Figs. 5A-5B, the examinee 30 was placed in a prone position. At the positions of the pubic bone portion and bone spur in the foregut, the pubic bone portion and bone spur in the foregut are pulled upward using the pillow 91 which assumes a horseshoe shape within a plane and which is mounted around the lumbar region. The anterior abdominal wall portion 64 is made to descend naturally. Under this state, the colonoscope 13 is inserted.
As described previously, in a prone position, the public bone portion is elevated, and the anterior abdominal wall portion is made to descend naturally. Thus, it is swollen downwardly. The sigmoid colon region 38 is collected at the bottom of the anterior abdominal wall portion 64 by the weight of the sigmoid colon region 38 itself and by the weight of the intestinal juice.
Thus, the sigmoid colon region 38 which is arranged like a very sharp hairpin curve and into which the colonoscope 13 is inserted with the greatest difficulty can be divided into a lower sigmoid colon region 88 directed toward the head direction from the rectal region 42 and an upper sigmoid colon region 89 that is inverted from the front end of the lower sigmoid colon region 88 and reaches the sigmoid colon-descending colon junction region 47.

As shown in Figs. 5A-5B, the tubular body portion 11 in which the colonoscope 13 has been previously inserted is inserted into the rectoanal region 12, the balloon body 27 mounted in the tubular body portion 11 is swollen, and the tubular body portion 11 is held into the rectoanal region 12.
The tubular body portion 11 may be held into the rectoanal region 12 before the colonoscope 13 is inserted into the rectoanal region 12.

As shown in Figs. 5A-5B, the colonoscope 13 inserted into the rectoanal region 12 passes beyond the rectal region 42 of the examinee 30, is bent by the resistance of the resurrection bone, and goes in the downward (downangle) direction.

The front-end portion 39 of the colonoscope 13 inserted in the large intestine 57 passes beyond the urinary bladder (for a male) or beyond both urinary bladder and womb (for a female) and is made to naturally slip down into the lower sigmoid colon region 88 that is in the anterior abdominal wall portion 64 by its own weight. The front-end portion 39 of the colonoscope 13 abuts against the anterior abdominal wall portion 64. The front-end portion 39 of the colonoscope 13 is naturally turned into the direction (upangle direction) directed toward the head by the rigidity resistance of the anterior abdominal wall portion 64.

In this way, the colonoscope 13 is inserted into the inside 57 of the large intestine more safely by allowing the colonoscope 13 to drop and slip down into the inside 57 of the large intestine by the weight of the scope itself than by pushing the scope into the large intestine. An accident consisting of perforation of the large intestine due to insertion of the colonoscope 13 can be effectively prevented.

Where it is difficult to check the direction of movement of the colonoscope 13 inside the large intestine, transparent water is made to flow down into the inner wall 15 of the large intestine from the clamp port 61 formed at the front-end portion 39 of the colonoscope 13, permitting one to check the direction of movement.

The direction of movement of the colonoscope 13 can be checked by inserting the colonoscope 13 in the direction in which the transparent water moves.

A field of view for the front-end portion 39 of the colonoscope 13 can be secured by injecting intestinal juice into the inside 57 of the large intestine and, if necessary, transparent water when the colonoscope 13 is inserted. The visibility of the top portion of the sigmoid colon region 39 can be maintained. Where the amount of urine remaining in the bladder of the person is large due to prostate hypertrophy or the like, the scope can pass beyond the sigmoid colon region 39 with greater ease by reducing the amount of remaining urine sufficiently even using catheterization.

As shown in Figs. 6A-6B, the air in the inside 57 of the large intestine is made to move more easily toward the rectoanal region 12 placed at a higher position than the abdominal region by depressurizing the inside 57 of the large intestine from a side of the rectoanal region 12, using the colonoscopic auxiliary tool 10. The air in the inside 57 of the large intestine can be forcedly and quickly expelled by suction from the tubular body portion 11.

Therefore, as shown in Figs. 6A-6B, after inserting the front-end portion 39 of the colonoscope 13 into the anterior abdominal wall portion 64, the negative pressure supply portion 21 is driven to draw in the air in the inside 57 of the large intestine from the plural hole portions 18 formed in the front-end portion 29 of the tubular body portion 11 via the negative pressure supply passages 20, thus depressurizing the inside 57 of the large intestine.

Suction from the rectoanal region 12 is performed using the colonoscopic auxiliary tool 10. The colonoscope 13 is rotated to the right while drawing in air even from the air intake hole 41 formed in the front-end portion 39 of the colonoscope 13. During this process, the colonoscope 13 is dragged up to a position inwardly spaced about 10 cm from the rectoanal region 12 at which the colonoscope 13 is about to come off from the rectoanal region 12. The sigmoid colon region 38 is folded like bellows and shortened.

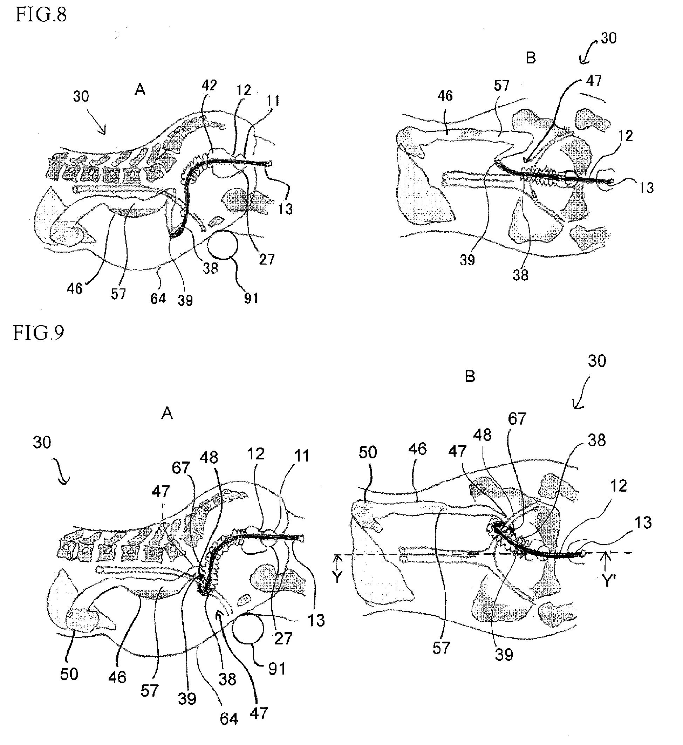

As shown in Figs. 7A-7B, the manipulation of the tubular body portion 11 for depressurization brings the inner wall 15 of the large intestine into intimate contact with the whole of the outer surface portion 56 of the cylindrical portion of the colonoscope 13. A large frictional force can be produced between the inner wall 15 of the large intestine and the outer surface portion 56 of the cylindrical portion of the scope. The sigmoid colon region 38 can be folded like bellows and shortened by dragging the colonoscope 13 toward the rectoanal region 12 while the frictional force is present.

Then, as shown in Figs. 8A-8B, suction and depressurization from the tubular body portion 11 is halted by manipulating an operation switch (not shown) mounted on the negative pressure supply portion 21. Depressurization inside the large intestine is weakened by stopping the suction from the front-end portion 39 of the colonoscope 13. Under this condition, the front-end portion 39 of the colonoscope 13 is made to fall into the anterior abdominal wall portion 64 again.

The folded large intestine can be held on the side of the rectoanal region 12 and disengagement of the inner wall 15 of the large intestine once brought into contact can be prevented by suction from the tubular body portion 11. Furthermore, the colonoscope 13 can be inserted while securing a field of view of the front-end portion 39 of the colonoscope 13 because suction is stopped in the front-end portion 39 of the colonoscope 13.

Where there is no strong colonic adhesion, the sigmoid colon region 38 can be shortened maximally by repeating the manipulations for drawing in the inside of the large intestine, shortening the large intestine while rotating the colonoscope to the right, and making the colonoscope drop down.

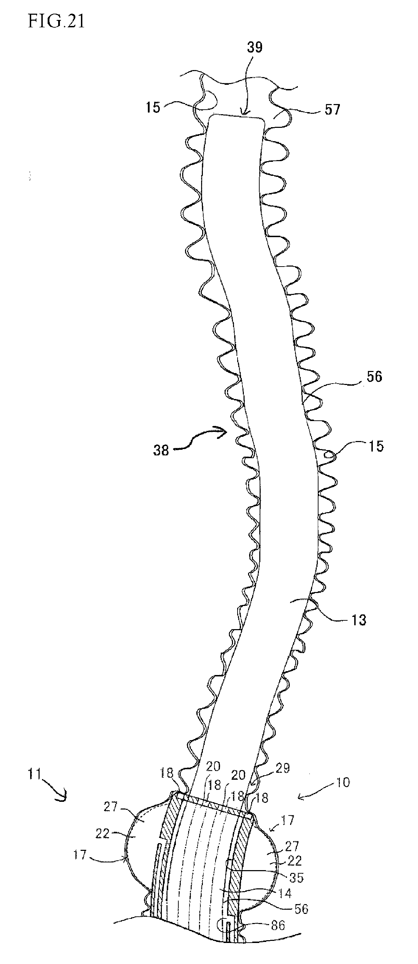

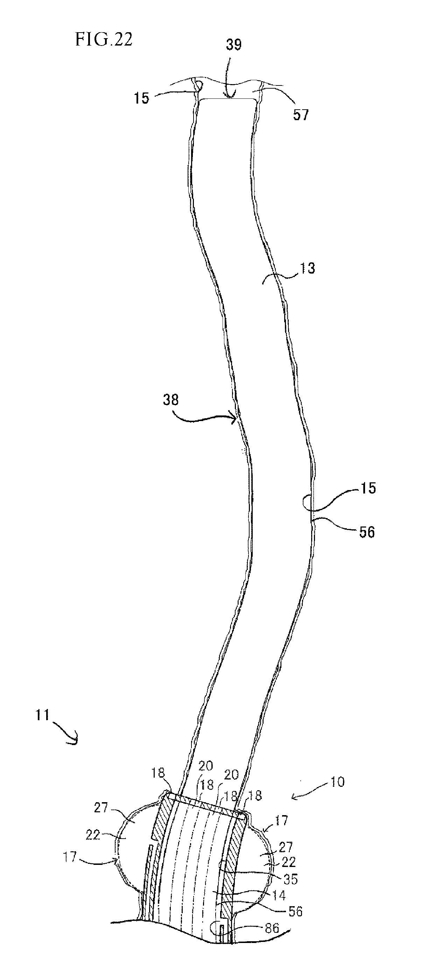

The work for inserting the colonoscope 13 into the inside 57 of the large intestine while performing the work for folding the large intestine by the above-described suction and depressurization is described in detail. As shown in Fig. 21, the colonoscope 13 is inserted into the inside 57 of the large intestine, and the inside 57 of the large intestine is depressurized by performing suction from the tubular body portion 11 mounted in the rectoanal region 12. As shown in Fig. 22, the inner wall 15 of the large intestine makes intimate contact with the whole of the outer surface portion 56 of the cylindrical portion of the colonoscope 13.

As shown in Fig. 22, with respect to the adhesion between the inner wall 15 of the large intestine and the outer surface portion 56 of the cylindrical portion of the scope, intimate contact is made over the whole of the outer surface portion 56 of the cylindrical portion of the scope as well as at the front-end portion 39 of the colonoscope 13. Therefore, larger frictional force is produced between the inner wall 15 of the large intestine and the outer surface portion 56 of the cylindrical portion of the scope than where suction only to the surroundings of the front-end portion 39 is performed.

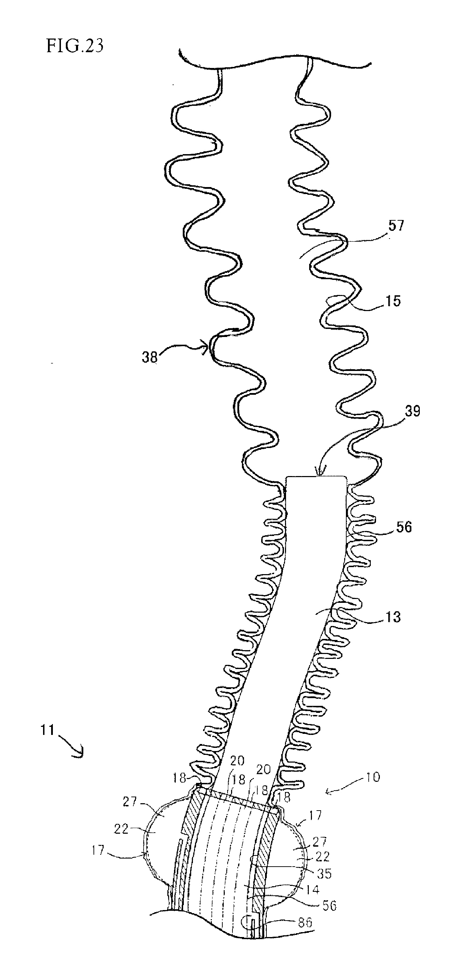

Accordingly, as shown in Fig. 23, suction from the rectoanal region 12 by means of the tubular body portion 11 produces a frictional force on the inner wall 15 of the large intestine and over the whole of the outer surface portion 56 of the cylindrical portion of the scope. Under this condition, the large intestine (e.g., the sigmoid colon region 38) can be folded like bellows by dragging the colonoscope 13 toward the rectoanal region 12.

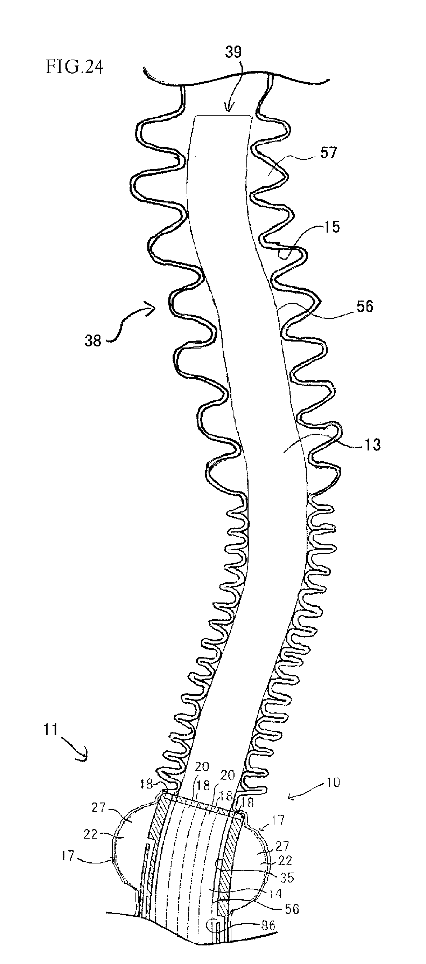

Then, as shown in Fig. 24, suction from the front-end portion 39 of the colonoscope 13 is stopped without stopping the suction and depressurization from the rectoanal region 12 by means of the tubular body portion 11. The front-end portion 39 of the colonoscope 13 is again inserted into the inside 57 of the large intestine.

As shown in Fig. 24, a field of view of the front-end portion 39 of the colonoscope 13 can be secured by stopping the suction from the front-end portion 39 of the colonoscope 13. Also, suction from the rectoanal region 12 is performed. Therefore, on the side of the rectoanal region 12, the state in which the large intestine has been folded like bellows can be maintained.

Therefore, the colonoscope 13 can be inserted further into the inside 57 of the large intestine while reliably performing folding of the large intestine (e.g., the sigmoid colon region 38) . Furthermore, a colonoscopic examination can be performed safely while securing a field of view of the colonoscope 13.

As shown in Figs. 9A-9B, the scope 13 is rotated in the rightward direction while sucking and depressurizing the inside 57 of the large intestine from the tubular body portion 11. The front-end portion 39 is placed in its neutral position. Under this condition, the colonoscope 13 is dragged up to about 25 cm from the rectoanal region 12. Thus, the front-end portion 39 is made to slip down into the left iliac cavity 67. The front-end portion 39 of the colonoscope 13 is caught on a dam-like portion (hereinafter abbreviated iliac arch 48 (this term "iliac arch" has been coined by the present inventor)) formed by joining those which branch into common iliac arteries and veins of the aorta and descending aorta into the iliac muscles.

As shown in Figs. 9A-9B, the colonoscope 13 is inserted while deaerating the inside 57 of the large intestine. This is achieved by performing suction from the front-end portion 39 of the colonoscope 13 and by suction from the tubular body portion 11 disposed on the side of the rectoanal region 12 in such a way that the inner cavity of the sigmoid colon region 38 is not stretched. Because of the resistance of the abdominal wall and liver portion, the front-end portion 39 comes close to the sigmoid colon-descending colon junction region 47.

The inner wall 15 of the large intestine is brought into intimate contact with the outer surface portion 56 of the colonoscope by sucking the inside 57 of the large intestine thoroughly from the front-end portion 39 of the colonoscope 13 and tubular body portion 11. The colonoscope 13 is placed in an upward direction (Upangle state). The colonoscope 13 is dragged toward the rectoanal region 12 while rotating the scope to the right. The sigmoid colon region 38 is shortened while the front-end portion 39 is held by the rigidity resistance of the anterior abdominal wall portion 64.

As shown in Figs. 9A-9B, when the front-end portion 39 of the colonoscope 13 is in an upward direction (Upangle state), the scope is rotated to the right. When the front-end portion is in a downward direction (Downangle state), the intestinal canal 45 is dragged while rotating the scope to the left. These works are repeated. Thus, the front-end portion 39 of the colonoscope 13 is dragged to the iliac arch 48 to shorten the intestinal canal 45 like bellows.

As shown in Figs. 9A-9B, the colonoscope 13 is slipped into the internal cavity of the sigmoid colon region 38 while rotating the colonoscope 13 in the rightward direction.
Even where the colonoscope 13 is rotated, if the internal cavity of the sigmoid colon region 38 cannot be discovered, a small amount of air is sent from the front-end portion 39 of the scope 13, and the colonoscope 13 is inserted 25 cm into the large intestine. The large intestine is dragged while rotating the scope to the right to shorten the intestine. Consequently, the internal cavity can be discovered with ease.

As shown in Fig. 4, the sigmoid colon region 38 is a region whose surroundings are not held by surrounding muscle fibers and so on. The descending colon region 46 is a part that is partially held by surrounding muscle fibers and so on. Therefore, if the colonoscope 13 is simply inserted into the sigmoid colon region 38 until the scope reaches the region making a transition to the descending colon region 46 (hereinafter referred to as the sigmoid colon-descending colon junction region 47) from the sigmoid colon region 38, the colonoscope 13 cannot be easily passed into deep regions of the large intestine, because the sigmoid colon region 38 is elongated in the direction in which the colonoscope 13 is inserted.

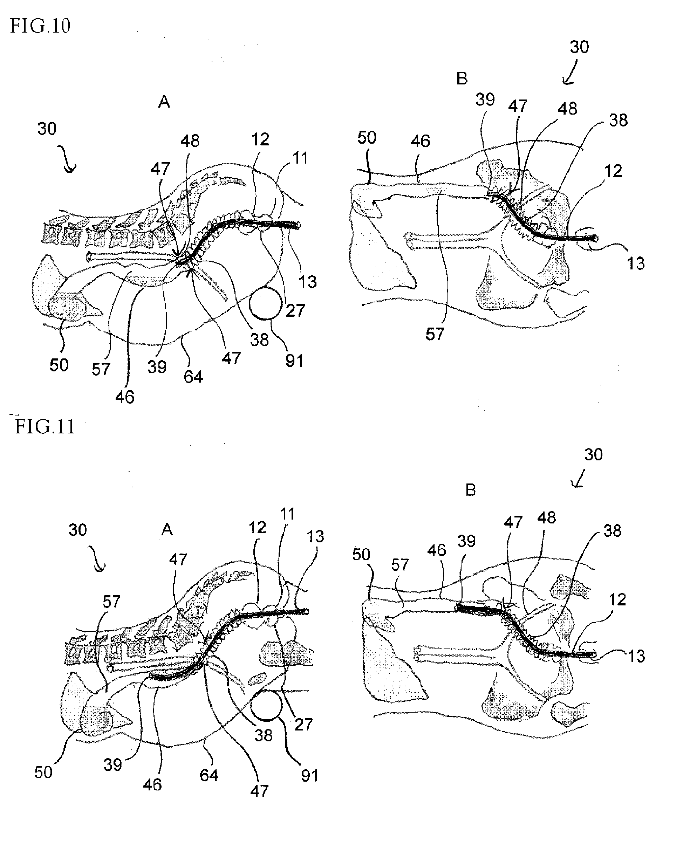

Accordingly, as shown in Figs. 10A-10B, the front-end portion 39 is caught on the iliac arch 48 by rotating the colonoscope 13 through 180 degrees in the rightward direction. The scope is rotated through 30 to 60 degrees in the rightward direction while depressurizing the inside 57 of the large intestine and maintaining dragging from the rectoanal region 12.
The internal cavity of the descending colon region 46 can be visually checked by placing the front-end portion 39 of the colonoscope 13 in its neutral position. It is possible that the colonoscope 13 will naturally slip into the descending colon region 46.

More specifically, as shown in Figs. 12A-12C, the front-end portion 39 of the colonoscope 13 is rotated through 30 to 60 degrees to the right while catching and supporting the front-end portion 39 of the colonoscope 13 on the iliac arch 48 that has been rigidified like a dam by the aorta, veins, iliac muscles, and so on. Thus, the sigmoid colon region 38 can be shortened like bellows and straightened.

Fig. 13 is a semi-cross-sectional model 70 taken by cutting the sigmoid colon region 38 of the examinee 30 in the direction of length in the central portion of the resurrection bone on Y-Y' cross section of Fig. 9 (referred to as a "sagittal section" in the field of the anatomy), showing a three-dimensional anatomical image of the colonic free space. A detailed description is given, using the human body model. As shown in Figs. 9 and 13, the front-end portion 39 of the colonoscope 13 inserted from the rectoanal region 12 is caught on the iliac arch 48 having relatively high rigidity and rotated through 30 to 60 degrees. Consequently, the front-end portion 39 can be inserted into the descending colon region 73.

As the sigmoid colon region 38 is straightened, the angle of refraction of the colonoscope 13 is made more obtuse. Therefore, the sigmoid colon region 38 is shortened further. The air inside the descending colon region 46 is moved toward the rectoanal region 12 by suction from the tubular body portion 11 and discharged to the outside from the tubular body portion 11.

As shown in Figs. 11A-11B, the front-end portion 39 of the colonoscope 13 is passed beyond the sigmoid colon-descending colon junction region47 by straightening the sigmoid colon region 38, and the inside cavity that has been split into two layers by the intestinal juice in the descending colon region 46 and air is made to appear in the field of view of the colonoscope 13. The front-end portion 39 is inserted into the descending colon region 46.

The colonoscope 13 is inserted about 10 cm into the inner cavity of the descending colon 46 while keep rotating the colonoscope 13 to the right without loosening the dragging of the large intestine and, at the same time, sucking the intestinal juice in the descending colon 46 from the front-end portion 39 of the colonoscope 13. Thus, the colonoscope 13 is moved through the descending colon region 46. As shown in Fig. 4, the front-end portion 39 of the colonoscope 13 reaches the splenic flexure region 50 formed between the transverse colon region 49 and the descending colon 46.

Also, in the case where the front-end portion 39 of the colonoscope 13 is made to reach the splenic flexure region 50, if the colonoscope 13 is pushed in, the colonoscope 13 can be inserted safely by slightly lowering the head side of the examination table carrying the examinee thereon, lowering the front-end portion 39 of the colonoscope 13, and permitting the colonoscope 13 to fall by its own weight because the sigmoid colon region 38 easily bends.

Then, as shown in Figs. 14-20, the process in which the colonoscope 13 goes from the transverse colon region 49 that is a free intestine to the cecal region 55 via the ascending colon region 54 that is a fixed intestine is described using a conceptual diagram using the model 99 of gypsum.

The sigmoid colon region 38 is straightened by performing the dragging and shortening work for the sigmoid colon region 38. Even where the front-end portion 39 goes beyond the splenic flexure region 50, if the front-end portion 39 of the colonoscope 13 is inserted into the transverse colon region 49, the transverse colon region 49 that is a free intestine is stretched in the direction in which the colonoscope 13 is inserted and, therefore, it becomes difficult to insert the colonoscope 13.
Therefore, a work for folding and straightening the transverse colon region 49 is necessary also for the transverse colon region 49 subsequently to the sigmoid colon region 38.

As shown in Figs. 14A-14C, in a case where the front-end portion 39 of the colonoscope 13 goes beyond the splenic flexure region 50 and the transverse colon region 49 is folded, the rectoanal region 12 is placed at the highest position and the splenic flexure region 50 is placed at a lower position by causing the left side of the body of the examinee 30 in a prone position to be elevated 45 degrees and letting the head of the examinee 30 sink.

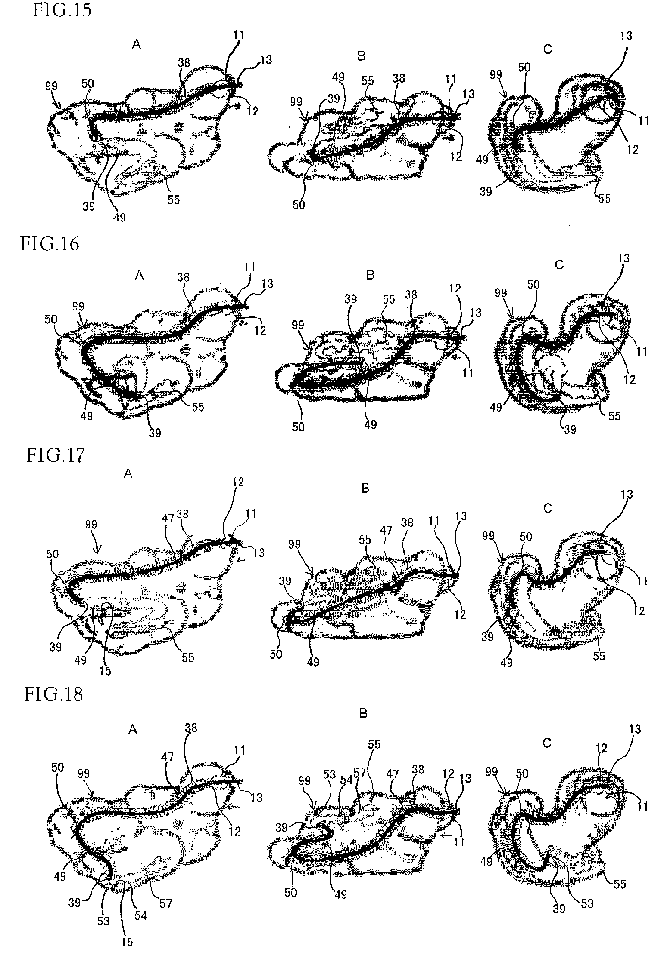

As shown in Figs. 15A-15C, the colonoscope 13 is dragged by applying a negative pressure from the tubular body portion 11 mounted at a side of the rectoanal region 12 under this condition. Thus, the transverse colon region 49 can be readily folded up.
In this case, the splenic flexure region 50 is a part of the fixed intestine but not firmly held to the abdominal cavity. Therefore, the splenic flexure region is slightly pulled toward the rectoanal region 12 by the dragging of the colonoscope 13.

As shown in Figs. 16A-16C, the examinee 30 is placed in a prone position close to the right prone position. Under this condition, the inside 57 of the large intestine is depressurized by sucking the air going upward toward the rectoanal region 12 by the tubular body portion 11. This is achieved by sucking the air inside the transverse colon region 49 from the tubular body portion 11 installed in the rectoanal region 12. Suction of the tubular body portion 11 disposed in the rectoanal region 12 is performed while bringing the inner wall 15 of the large intestine of the transverse colon region 49 into intimate contact with the outer surface portion 56 of the colonoscope. Thus, the colonoscope 13 is pulled into the inside 57 of the large intestine by the weight of the colonoscope 13 itself and by the negative pressure in the inside 57 of the large intestine. Therefore, the colonoscope 13 can be inserted into the transverse colon region 49 while preventing the straightened sigmoid colon region 38 from being returned to the original bent state.

As shown in Figs. 17A-17C, the transverse colon region 49 that is a free intestine is folded like bellows and the transverse colon region 49 is shortened by dragging the inserted colonoscope 13 toward the rectoanal region 12.

Then, as shown in Figs. 18A-18C, in a case where the hepatic flexure region 53 is seen in the front-end portion 39 of the colonoscope 13 and the front-end portion 39 then passes beyond the hepatic flexure region 53, the colonoscope 13 can be easily inserted into the ascending colon region 54. In this case, insertion can be facilitated further by letting the examinee 30 lower the head portion. The air inside the ascending colon region 54 is sucked by suction from the tubular body portion 11 disposed on a side of the rectoanal region 12, thus depressurizing the inside of the ascending colon region 54.

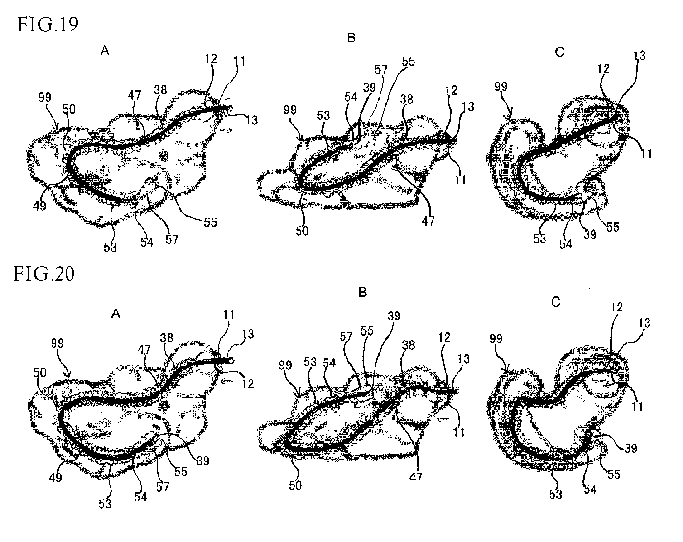

Because of the above-described depressurization, the transverse colon region 49 can be folded like bellows as shown in Figs. 19A-19C by dragging the colonoscope 13 toward the rectoanal region 12 while bringing the inner wall 15 of the large intestine of the ascending colon region 54 into intimate contact with the outer surface portion 56 of the colonoscope. The transverse colon region 49 can be raised toward the rectoanal region 12 and the transverse colon region 49 can be shortened.
The colonoscope 13 is made to drop into the ascending colon region 54 by its own weight. The inside of the ascending colon region 54 is in a negative pressure state because of suction from the tubular body portion 11.

Accordingly, the straightened state of the sigmoid colon region 38 is maintained by repeating the operations for suction and depressurization as shown in Figs. 20A-20C. The doctor can insert the colonoscope 13 up to the cecal region 55 unconsciously of the hepatic flexure region 53 without pushing in the colonoscope 13, by permitting the colonoscope 13 to drop into the inside 57 of the large intestine by the weight of the colonoscope 13 itself.

The colonoscopic auxiliary tool associated with the present invention is not limited to the above embodiment. The negative pressure supply passages 20 in the tubular body portion 11 can be omitted by fabricating the material (not shown) of the tubular body portion 11 from a high molecular material or the like that can pass only gas from the side of the front-end portion 29 of the tubular body portion 11 to the side of the base portion 28.
In this case, the air inside the large intestine can be sucked from the whole front-end portion of the tubular body portion by means of the negative pressure supply portion 21 mounted in the base portion 28 of the tubular body portion 11.
Accordingly, the inside of the large intestine can be depressurized by inserting the tubular body portion 11 into the rectoanal region and sucking the air inside the large intestine from the whole front-end portion of the tubular body portion 11. Works for shortening and folding the large intestine can be performed.

### INDUSTRIAL APPLICABILITY

The invention of the present application can be used as a colonoscopic auxiliary tool used when a colonoscopic examination is performed, and can be applied to a colonoscopic auxiliary tool with which an inspecting doctor can easily perform a colonoscopic examination without requiring long-term training.

### BRIEF DESCRIPTION OF THE DRAWINGS

[Fig. 1] Fig. 1 is a sideways cross section showing a colonoscopic auxiliary tool associated with the present invention.
[Fig. 2] Fig. 2 is a lower plan view under the state in which a cover portion showing a colonoscopic auxiliary tool associated with the present invention is seen through.
[Fig. 3] Fig. 3 is a perspective view showing the state in which a colonoscopic auxiliary tool associated with the present invention is installed in an examinee and a colonoscope has been inserted.
[Fig. 4] Fig. 4 is a conceptual diagram showing a general intestinal canal structure of an examinee.
[Fig. 5] Figs. 5A-5B are perspective views showing the state in which a front-end portion of a colonoscope abuts against the anterior abdominal wall portion.
[Fig. 6] Figs. 6A-6B are perspective views showing the state in which the inside of the large intestine has been depressurized in the state shown in Fig. 5 and the inner wall of the large intestine has been brought into intimate contact with the outer surface portion of the cylindrical portion of the scope.
[Fig. 7] Figs. 7A-7B are perspective views showing the state in which the large intestine in the state shown in Fig. 6 has been dragged and folded like bellows.
[Fig. 8] Figs. 8A-8B are perspective views showing the state in which the colonoscope is again inserted under the state in which suction from the tubular body portion is halted by manipulating an operation switch in the state shown in Fig. 7 and the depressurizing work has been stopped.
[Fig. 9] Figs. 9A-9B are perspective views showing the state in which a colonoscopic auxiliary tool associated with the present invention has been held to the rectoanal region and the front-end portion of the scope has been caught on an iliac arch.
[Fig. 10] Figs. 10A-10B are perspective views showing the state in which a colonoscopic auxiliary tool associated with the present invention has been held to the rectoanal region, the inside cavity of the descending colon region can be visually checked by placing the front-end portion of the scope in its neutral position while rotating the scope to the right in the sigmoid colon region and, under this condition, the scope has been naturally slipped into the descending colon region.
[Fig. 11] Figs. 11A-11B are perspective views showing the state in which the front-end portion of a colonoscope has been inserted into the descending colon region beyond the sigmoid colon-descending colon junction region.
[Fig. 12] Figs. 12A-12C are sideways perspective views showing the state in which a colonoscopic auxiliary tool associated with the present invention has been held to the rectoanal region, the front-end portion of the scope has been caught on an iliac arch, and the colonoscope has been rotated through 30 to 60 degrees to the right.
[Fig. 13] Fig. 13 is a semi-cross-sectional model showing a three-dimensional anatomical image in a colonic free space, taken by cutting the central portion of the resurrection bone in the direction of length along Y-Y' cross section of Fig. 9 (anatomically referred to as a "sagittal section").
[Fig. 14] Figs. 14A-14C are perspective views showing the state in which a colonoscopic auxiliary tool associated with the present invention has been held to the rectoanal region and the front-end portion of the scope has passed beyond the splenic flexure region.
[Fig. 15] Figs. 15A-15C are perspective views showing the state in which a colonoscopic auxiliary tool associated with the present invention has been held to the rectoanal region, the front-end portion of the scope has been inserted in the transverse colon region, and the large intestine has been folded up by depressurizing the inside of the large intestine and dragging the colonoscope.
[Fig. 16] Figs. 16A-16C are perspective views showing the state in which the colonoscope in the state shown in Fig. 15 has been inserted into the transverse colon region by the weight of the colonoscope itself and by a negative pressure inside the large intestine.
[Fig. 17] Figs. 17A-17C are perspective views showing the state in which the transverse colon region has been folded like bellows by depressurizing the inside of the large intestine in the state shown in Fig. 16 and dragging the colonoscope.
[Fig. 18] Figs. 18A-18C are perspective views showing the state in which a colonoscopic auxiliary tool associated with the present invention has been held to the rectoanal region and the hepatic flexure region is visible in the front-end portion of the scope.
[Fig. 19] Figs. 19A-19C are perspective views showing the state in which a colonoscopic auxiliary tool associated with the present invention has been held to the rectoanal region and the transverse colon region has been folded up while sucking the air inside the ascending colon.
[Fig. 20] Figs. 20A-20C are perspective views showing the state in which a colonoscopic auxiliary tool associated with the present invention has been held to the rectoanal region and the front-end portion of the colonoscope has been made to reach the cecal region by inserting the scope while depressurizing the inside of the ascending colon.
[Fig. 21] Fig. 21 is a planar perspective view showing the state in which a colonoscopic auxiliary tool associated with the present invention has been held to the rectoanal region and the colonoscope has been inserted in the large intestine.
[Fig. 22] Fig. 22 is a planar perspective view showing the state in which the inside of the large intestine in the state shown in Fig. 21 has been depressurized and the inner wall of the large intestine has been brought into intimate contact with the outer surface portion of the cylindrical portion of the scope.
[Fig. 23] Fig. 23 is a planar perspective view showing the state in which the large intestine in the state shown in Fig. 22 has been folded like bellows by dragging the large intestine. [Fig. 24] Fig. 24 is a perspective view showing the state in which the colonoscope in the state shown in Fig. 23 has been again inserted without stopping suction from the tubular body portion.
[Fig. 25] Fig. 25 is a plan view showing one embodiment of a front-end portion of a scope used in a colonoscopic auxiliary tool associated with the present invention.
[Fig. 26] Fig. 26 is a side elevation showing a model of gypsum created based on an image taken by CT scanning of a maximum movable range of the free intestines (sigmoid colon and transverse colon regions) inside the large intestine of the present inventor himself, depicting the state in which an examinee is placed in a prone position and the left abdominal wall has been raised 45 degrees.
[Fig. 27] Fig. 27 is a look-down view taken from the rear surface of an examinee under the state in which the examinee is placed in a prone position and the left abdominal wall has been raised 45 degrees, showing a model of gypsum created based on an image taken by CT scanning of a maximum movable range of the free intestines (sigmoid colon and transverse colon regions) inside the large intestine of the present inventor himself.
[Fig. 28] Fig. 28 is a view taken from the anal side of an examinee showing the state in which the examinee is placed in a prone position and the left abdominal wall has been raised 45 degrees, showing a model of gypsum created based on an image taken by CT scanning of a maximum movable range of the free intestines (sigmoid colon and transverse colon regions) inside the large intestine of the present inventor himself.

### DESCRIPTION OF REFERENCE NUMERALS AND SIGNS

- 10:: colonoscopic auxiliary tool
- 11:: tubular body portion
- 12:: rectoanal region
- 13:: colonoscope
- 14:: cylindrical portion of the scope
- 15:: inner wall of the large intestine
- 16:: suction-and-depressurization portion
- 17:: holding portion
- 18:: hole portions
- 19:: depressurization mechanism
- 20:: negative pressure supply passages
- 21:: negative pressure supply portion
- 22:: expansible portion
- 23:: fluid supply portion
- 25:: lubricant supply portion
- 26:: air inflow cutoff portion
- 27:: balloon body
- 28:: base portion
- 29:: front-end portion
- 30:: examinee
- 33:: fluid supply passage
- 34:: lubricant supply passage
- 35:: peripheral surface of inner wall
- 36:: adhesive tape
- 37:: fluid supply source
- 38:: sigmoid colon region
- 39:: front-end portion
- 40:: air-feeding hole
- 41:: air intake hole
- 42:: rectal region
- 43:: left abdominal wall
- 45:: intestinal canal
- 46:: descending colon region
- 47:: sigmoid colon-descending colon junction region
- 48:: iliac arch
- 49:: transverse colon region
- 50:: splenic flexure region
- 51:: suction port of front-end portion
- 52:: suction port of side surface portion
- 53:: hepatic flexure region
- 54:: ascending colon region
- 55:: cecal region
- 56:: outer surface portion of cylindrical portion of scope
- 57:: inside of the large intestine
- 58:: lubricant supply source
- 59:: observational lens
- 60:: light guide
- 61:: clamp port
- 64:: anterior abdominal wall portion
- 67:: left iliac cavity
- 69:: negative pressure supply hole portion
- 70:: model
- 80:: hoses
- 81:: fluid supply hole portion
- 82:: hose
- 83:: lubricant supply hole portion
- 85:: hose
- 86:: opening portion
- 88:: lower sigmoid colon region
- 89:: upper sigmoid colon region
- 90:: holding element
- 91:: pillow
- 92:: negative pressure chamber
- 93:: cover portion
- 94:: (larger) adapter
- 95:: (smaller) adapter
- 96:: air inflow cutoff portion
- 97:: check valve
- 98:: check valve
- 99:: model of gypsum

## Claims

1. A colonoscopic auxiliary tool for use with a colonoscope during a colonoscopic examination, the auxiliary tool comprising:
a tubular body portion which is disposed in a rectoanal region and through which the colonoscope is passed; and
a suction-and-depressurization portion mounted in the tubular body portion, the suction-and-depressurization portion being capable of depressurizing inside of a large intestine from the rectoanal region to bring an inner wall of the large intestine into contact with an outer surface portion of a cylindrical portion of the colonoscope.

2. A colonoscopic auxiliary tool as set forth in claim 1,
wherein the suction-and-depressurization portion has a plurality of hole portions formed in the tubular body portion and a depressurization mechanism capable of sucking air inside the large intestine from the hole portions via the plural hole portions to form a negative pressure inside the large intestine, and
wherein the depressurization mechanism has negative pressure supply passages in communication with the plural hole portions and a negative pressure supply portion capable of supplying a negative pressure via the negative pressure supply passages.

3. A colonoscopic auxiliary tool as set forth in claim 1 or claim 2, wherein a lubricant supply portion capable of supplying a lubricant into the tubular body portion is mounted in the tubular body portion such that the scope is smoothly inserted into the tubular body portion.

4. A colonoscopic auxiliary tool as set forth in claim 1 to 3, wherein an air inflow cutoff portion capable of preventing inflow of air from the outside into the tubular body portion is mounted on a base portion side of the tubular body portion and pressed against the inserted colonoscope.

5. A colonoscopic auxiliary tool as set forth in claim 1 or claim 2, comprising a holding portion capable of holding the tubular body portion into the rectoanal region.

6. A colonoscopic auxiliary tool as set forth in claim 5, wherein the holding portion has an expansible portion mounted outside the tubular body portion and being capable of swelling outwardly of the tubular body portion by being supplied with a fluid and a fluid supply portion capable of supplying the fluid to the expansible portion.

7. A colonoscopic auxiliary tool as set forth in claim 6, wherein the expansible portion is made of a balloon body which is mounted in a front-end portion of the tubular body portion and which swells out by being supplied with the fluid.

8. A colonoscopic auxiliary tool as set forth in claim 7, wherein the balloon body is mounted over the whole periphery of the tubular body portion.

9. A colonoscopic auxiliary tool as set forth in claim 1 to 4, wherein holding elements capable of supporting the tubular body portion outside the anal region are mounted on a base portion side of the tubular body portion.
